# FASCICULE DE BREVET EUROPEEN

(11) **EP 2 398 411 B1**
(45) Date de publication et mention de la délivrance du brevet: **30.04.2014**
(21) Numéro de dépôt: 10708241.4
(22) Date de dépôt: 17.02.2010
(51) Int. Cl.: A61B 17/80, A61B 17/00, A61B 19/00

(54) **ENSEMBLE SUR MESURE D'AU MOINS DEUX PLAQUES D'OSTEOSYNTHESE, ELLES-MEMES PREPAREES SUR MESURE**
PATIENTENSPEZIFISCHE ANORDNUNG MIT MINDESTENS ZWEI IHRERSEITS PATIENTENSPEZIFISCH AUSGELEGTEN OSTEOSYNTHESEPLATTEN
CUSTOMISED ASSEMBLY INCLUDING AT LEAST TWO OSTEOSYNTHESIS PLATES WHICH ARE IN TURN CUSTOMISED

(30) Priorité: 17.02.2009 FR 0900722
(43) Date de publication de la demande: 28.12.2011
(60) Demande divisionnaire: 14161014.7
(73) Titulaire: OBL, 92320 Chatillon (FR)
(72) Inventeur: LONGEPIED, Patrice, F-92330 Sceaux (FR)
(74) Mandataire: Blaseby, Matthew Peter
(86) Numéro de dépôt international: PCT/FR2010/000134
(87) Numéro de publication internationale: WO 2010/094857

(56) Documents cités:
- EP-A- 1 468 656
- FR-A- 2 626 460
- US-A1- 2002 128 654

## Description

La présente invention est relative à un ensemble sur mesure d'au moins deux plaques d'ostéosynthèse, par exemple de types droites en I et/ou en L et/ou en croix et/ou en forme de patte-d'oie, elles-mêmes préparées sur mesure en fonction, d'une part, de l'opération à réaliser sur une mâchoire ou sur les deux mâchoires d'un patient et, d'autre part, de la future anatomie dudit patient.

Elle a également trait à un procédé de mise en place dudit ensemble sur mesure sur le patient.

On sait que, dans le cadre de la chirurgie plastique, en particulier orthognatique et traumatique, ou encore lors de certaines interventions chirurgicales, et notamment lors d'une intervention d'ostéosynthèse maxillomandibulaire - que cette dernière ne modifie pas l'occlusion ou qu'elle ne modifie pas les rapports occlusiaux ou encore qu'elle soit totale, en modifiant une arcade alvéolaire, ou segmentaire en n'intéressant qu'une partie de l'arcade - il est toujours nécessaire de recourir à la pose de plaques d'ostéosynthèse, c'est-à-dire de plaques de fixation vissées entre les parties osseuses qui se trouvent alors disjointes.

De telles plaques sont bien connues, en particulier du brevet français publié sous le n° 2.531.855 ou encore du brevet français publié sous le n° 2.725.124. EP 1468656 A décrit un ensemble selon le préambule de la revendication 1.

Ces plaques d'ostéosynthèse, qui sont le plus souvent réalisées en titane, sont tordues à la main par le chirurgien, avec des pinces, afin qu'elles s'adaptent parfaitement à la future anatomie du patient.

On sait par ailleurs réaliser, à partir des données d'un scanner ou d'une IRM, la simulation de l'ostéotomie grâce à laquelle on peut, au choix, avancer ou reculer la pièce osseuse du maxillaire et/ou de la mandibule jusqu'à, comme cela est souhaité, obtenir la disposition des dents du patient en regard les unes des autres.

Avant de procéder à une telle opération d'occlusion dentaire, on sait simuler par avance les résultats de l'opération sur le patient et l'on peut en conséquence préparer sur mesure des plaques d'ostéosynthèse de formes diverses qui devront être vissées aux endroits appropriés dans les éléments osseux d'une ou des deux mâchoires du patient, jusqu'à cicatrisation de l'os.

Cette préparation va jusqu'à donner elle aussi, ainsi qu'il a été dit en préliminaire, toutes les courbures voulues aux plaques d'ostéosynthèse, et ce afin d'adapter parfaitement ces dernières à la future anatomie du patient.

Cela étant, et ce problème n'a pas encore été résolu, rien ne permet d'assurer que le chirurgien placera exactement aux bons endroits, sur la ou sur les deux mâchoires du patient, les plaques d'ostéosynthèse préparées sur mesure par avance.

La présente invention a pour but d'apporter une solution à ce problème en proposant un ensemble sur mesure d'au moins deux plaques d'ostéosynthèse, elles-mêmes préparées sur mesure.

Une telle structure d'ensemble se caractérise par le fait que les plaques d'ostéosynthèse sont reliées entre elles par une tige, laquelle tige comporte en outre deux proéminences qui émergent de ladite tige en direction de l'orifice nasal du patient en des positions qui correspondent aux deux extrémités de la base de l'orifice nasal du patient.

Les coins inférieurs de l'orifice nasal du patient ne pouvant varier en position, le fait pour le chirurgien de les utiliser comme références naturelles pour loger les proéminences de la structure d'ensemble selon l'invention et, à cette suite, pour placer ladite structure d'ensemble soit sur la mâchoire inférieure, soit sur la mâchoire supérieure du patient concernée par l'opération, garantit que toutes les plaques d'ostéosynthèse de ladite structure occuperont sans aucune exception les emplacements qu'il a par avance été prévu qu'elles tiennent.

Avantageusement, les deux proéminences ont chacune la forme d'un téton, d'un doigt, d'un ergot ou d'un éperon.

Les deux repères en saillie ainsi prévus par construction sur la tige de la structure d'ensemble selon l'invention étant des proéminences qui viennent se nicher dans les coins inférieurs de l'orifice nasal du patient, la pose par le chirurgien de ladite structure d'ensemble, avec la plus grande précision qui soit, en est facilité.

Toujours avantageusement, la tige de la structure d'ensemble selon l'invention respecte elle-aussi parfaitement l'anatomie du patient.

Dans une autre forme préférée de réalisation, la structure d'ensemble selon l'invention se caractérise par le fait que, à proximité de chacune de ses plaques, la tige de ladite structure présente des zones de moindre résistance destinées à faciliter la coupe par le chirurgien de toutes les parties de liaison entre les différentes plaques d'ostéosynthèse de ladite structure.

Après son placement en position idéale, par le chirurgien, de la structure d'ensemble selon l'invention, puis fixation de toutes les plaques d'ostéosynthèse par vissage dans l'os de la mâchoire supérieure ou de la mâchoire inférieure du patient, il est à l'évidence souhaitable de faire immédiatement disparaître toutes les parties de liaison entre lesdites plaques, y compris la partie qui présente les proéminences ayant permis la mise précise en position idéale de la structure d'ensemble sur la mâchoire du patient. Les zones de moindre résistance prévues à proximité immédiate des plaques ne feront que simplifier la tâche du chirurgien tout au long de cette opération d'élimination des parties de la structure d'ensemble selon l'invention devenues inutiles.

La structure d'ensemble selon l'invention est avantageusement fabriquée d'une seule pièce et, de préférence, elle est réalisée en titane.

La présente invention a également pour objet un procédé de mise en place d'au moins deux plaques d'ostéosynthèse sur le maxillaire et/ou sur la mandibule d'un patient, lors d'une opération d'occlusion dentaire, ledit procédé étant caractérisé en ce que, en préliminaire de ladite opération, l'on simule par avance les résultats de l'opération sur le patient, l'on détermine la position et la forme des plaques d'ostéosynthèse en fonction de la future anatomie du patient, l'on réalise sur mesure un ensemble d'au moins deux plaques d'ostéosynthèse, par exemple de types droites en I et/ou en L et/ou en croix et/ou en forme de patte-d'oie, elles-mêmes réalisées sur mesure en fonction de l'opération à réaliser et de la future anatomie du patient, ledit ensemble comprenant une tige qui relie entre elles les plaques d'ostéosynthèse et qui comporte en outre deux proéminences correspondant aux coins inférieurs de l'orifice nasal du patient, et en ce que, après l'opération, le chirurgien place idéalement ledit ensemble sur mesure en disposant ses deux proéminences dans les coins inférieurs de l'orifice nasal du patient, puis il fixe toutes les plaques d'ostéosynthèse à l'aide de vis pénétrant les éléments osseux de la mâchoire supérieure ou de la mâchoire inférieure concernée, puis il coupe à proximité de chacune des plaques d'ostéosynthèse dudit ensemble toutes les parties de liaison formées par la tige entre lesdites plaques.

L'invention sera mieux comprise à la lecture de la description qui va suivre, et en référence aux dessins annexés qui illustrent de manière non limitative un mode de réalisation de ladite invention, et sur lesquels :
- les Figures 1 et 2 représentent, vu de face et respectivement vu de côté, un exemple de réalisation de la structure d'ensemble sur mesure de plaques d'ostéosynthèse selon l'invention, ladite structure d'ensemble comportant quatre plaques en L dans un tel exemple.
- la Figure 3 illustre en vue de face le placement en position idéale sur la mâchoire supérieure d'un patient de l'ensemble sur mesure des Figures 1 et 2.
- la Figure 4 illustre en vue de profil le même placement en position idéale de l'ensemble sur mesure des Figures 1 et 2 sur la mâchoire supérieure du patient.

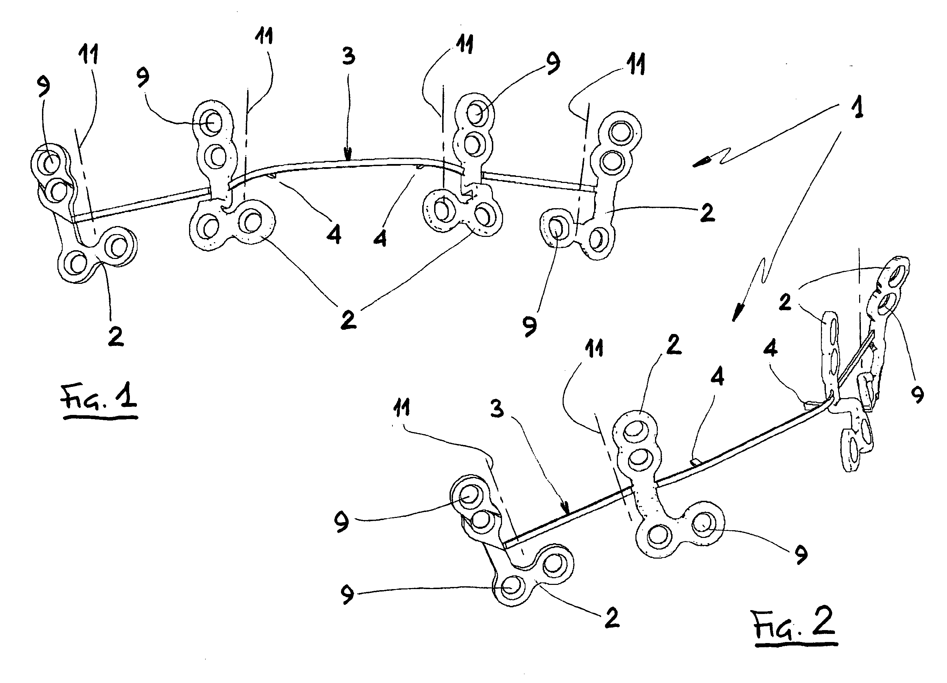

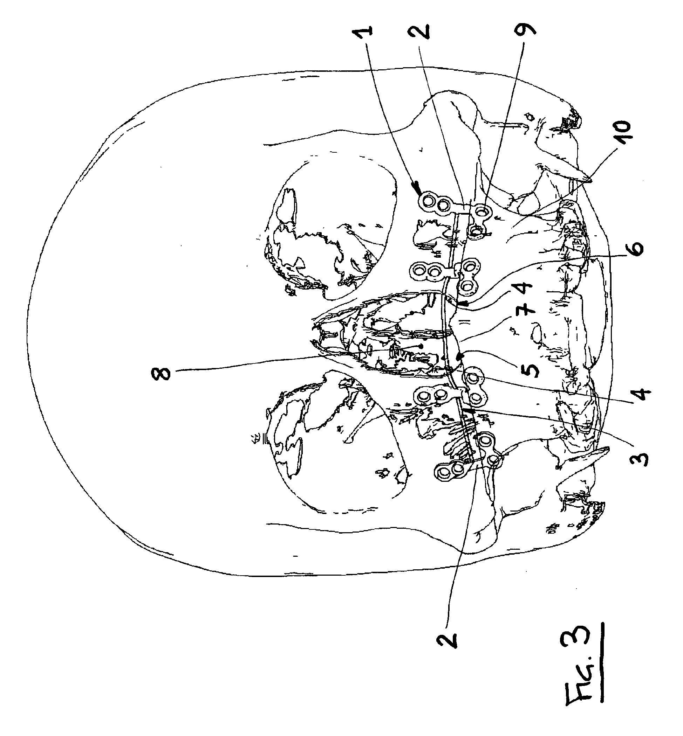

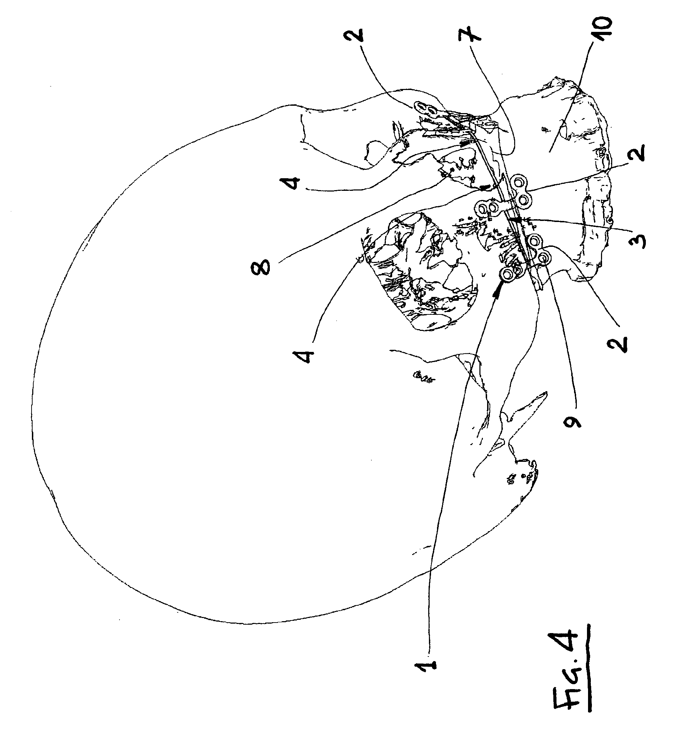

Dans l'exemple choisi, représenté sur les Figures 1 à 4, l'opération d'occlusion dentaire réalisée porte exclusivement sur la mâchoire supérieure, encore appelée maxillaire.

En variante, il est toutefois bien clair que l'invention s'applique également à toute réparation de la mâchoire inférieure, encore appelée mandibule, que cette réparation soit réalisée seule ou concomitamment avec celle de la mâchoire supérieure.

Dans cette variante, seules seront modifiées la forme de la tige de liaison ainsi que les formes des plaques d'ostéosynthèse qui, pour la réparation du maxillaire, sont le plus souvent du type en L et/ou en croix et/ou en forme de patte-d'oie.

En effet, pour la réparation de la mandibule, il leur est généralement préféré des plaques d'ostéosynthèse en forme de I, c'est-à-dire des plaques droites.

Connaissant la future anatomie du patient à partir des données d'un scanner ou d'une IRM, on réalise conformément à l'invention, en préliminaire de l'opération convenue, un ensemble 1 d'au moins deux plaques d'ostéosynthèse, quatre en L dans l'exemple représenté sur les Figures 1 à 4, qui est formé :
- des plaques d'ostéosynthèse 2 à proprement parler, lesquelles, en fonction précisément de la future anatomie du patient, et donc des positions qu'elles devront occuper sur sa mâchoire à réparer, sont mises chacune à la forme appropriée, c'est-à-dire sur mesure, avec ici et là les courbures qui leur garantiront d'épouser parfaitement la future anatomie désirée,
- une tige 3 qui relie entre elles toutes les plaques 2, ladite tige comportant en outre deux proéminences 4 correspondant très exactement aux deux extrémités, respectivement 5 et 6, de la base 7 de l'orifice nasal 8 du patient.

chaque plaque d'ostéosynthèse 2 est, de façon connue, percée de plusieurs trous traversants 9, ici quatre trous pour chaque plaque en forme de L, lesquels trous sont destinés à recevoir les vis qui, après la mise en place parfaite de l'ensemble 1 (encore appelé structure d'ensemble) sur le maxillaire 10 du patient, assureront la fixation dudit ensemble sur ledit maxillaire en étant vissées par le chirurgien dans les parties osseuses qui, disjointes après l'opération, doivent être réunies en étant placées les unes contre les autres.

Les deux proéminences 4 sont par exemple avantageusement en forme de tétons ou de doigts, ou d'ergots, ou d'éperons, lesquels émergent de la tige 3 en direction de l'orifice nasal 8 du patient. Toujours par construction, les positions sur la tige et les formes de ces proéminences 4 ont été déterminées à partir des données connues des scanners ou IRM.

De préférence, la tige 3 respecte elle-aussi parfaitement l'anatomie de l'os maxillaire ou, selon, celle de l'os mandibulaire du patient ; ses parties courbes et rectilignes sont donc connues à partir des données précitées et elles sont ainsi reproduites à l'identique sur la tige lors de la construction de la structure d'ensemble.

Après avoir effectué l'opération désirée, le chirurgien place les unes contre les autres les parties osseuses disjointes, puis il met en place la structure 1 en disposant les deux proéminences 4 dans les coins inférieurs 5 et 6 de l'orifice nasal 8 du patient, c'est-à-dire en les logeant aux deux extrémités de la base 7 dudit orifice nasal.

La structure 1 occupe idéalement sa place, déterminée à l'avance, par rapport au maxillaire du patient et le chirurgien n'a plus alors qu'à procéder à la fixation de ladite structure sur le maxillaire à l'aide des vis traversant les trous 9 des plaques d'ostéosynthèse 2 en L.

Les parties osseuses disjointes étant ainsi réunies fixement, et devant l'être jusqu'à la cicatrisation de l'os, le chirurgien n'a plus qu'à éliminer de la structure 1 toutes les parties de celle-ci devenues inutiles, c'est-à-dire les parties de liaison entre les différentes plaques d'ostéosynthèse 2, seules lesdites plaques demeurant donc dans la bouche du patient en fin d'opération.

Pour cela, le chirurgien coupe la tige 3 à proximité immédiate de chacune des plaques 2, y compris la partie de la tige qui comprend les deux proéminences 4.

Afin de faciliter ce travail de coupe de la tige en vue de son élimination, on prévoit par construction que ladite tige présente au voisinage de chacune des différentes plaques 2 des zones de moindre résistance illustrées par les traits mixtes 11, ces derniers, pour une meilleure clarté des dessins, n'ayant été schématisés qu'en quelques occasions.

L'ensemble sur mesure 1 selon l'invention - plaques d'ostéosynthèse 2 et tige 3 - est avantageusement fabriqué en titane, matériau biocompatible d'usage maintenant courant en chirurgie, et ledit ensemble est de préférence réalisé d'une seule pièce.

Comme il va de soi, l'invention ne se limite pas aux seules spécifications techniques données ci-dessus à titre d'exemples ; elle embrasse, au contraire, toutes les variantes possibles de réalisation, en particulier celle de la construction d'une autre structure 1 adaptée spécifiquement à la réparation d'une mandibule, autre structure pour laquelle, d'une part, la tige 3, au lieu d'être sensiblement rectiligne, aura davantage la forme d'un oméga et, d'autre part, des plaques 2 en forme de I viendront selon la tradition se substituer aux plaques choisies dans le présent exemple en forme de L, mais qui pourraient aussi bien être en croix ou en forme de patte-d'oie.

## Revendications

1. Ensemble sur mesure (1) d'au moins deux plaques d'ostéosynthèse (2), par exemple de types droites en I et/ou en L et/ou en croix et/ou en forme de patte-d'oie, elles-mêmes préparées sur mesure en fonction de l'opération à réaliser et de la future anatomie du patient, les plaques d'ostéosynthèse (2) étant reliées entre elles par une tige (3),
**caractérisé en ce que**
ladite tige comporte en outre deux proéminences (4) qui émergent de ladite tige (3) en direction de l'orifice nasal (8) du patient en des positions qui correspondent aux deux extrémités (5, 6) de la base (7) de l'orifice nasal (8) du patient.

2. Ensemble sur mesure (1) d'au moins deux plaques d'ostéosynthèse (2) selon la revendication 1, **caractérisé en ce que** les deux proéminences (4) ont la forme de tétons, ou de doigts, ou d'ergots, ou d'éperons.

3. Ensemble sur mesure (1) d'au moins deux plaques d'ostéosynthèse (2) selon l'une quelconque des revendications 1 et 2, **caractérisé en ce que** la tige (3) dudit ensemble respecte elle-aussi parfaitement l'anatomie du patient.

4. Ensemble sur mesure (1) d'au moins deux plaques d'ostéosynthèse (2) selon l'une quelconque des revendications 1 à 3, **caractérisé en ce que**, à proximité de chacune de ses plaques (2), la tige (3) dudit ensemble présente des zones de moindre résistance (11) destinées à faciliter la coupe par le chirurgien de toutes les parties de liaison entre les différentes plaques d'ostéosynthèse dudit ensemble.

5. Ensemble sur mesure (1) d'au moins deux plaques d'ostéosynthèse (2) selon l'une quelconque des revendications 1 à 4, **caractérisé en ce qu'**il est fabriqué d'une seule pièce.

6. Ensemble sur mesure (1) d'au moins deux plaques d'ostéosynthèse (2) selon l'une quelconque des revendications 1 à 5, **caractérisé en ce qu'**il est réalisé en titane.

## Patentansprüche

1. Angepasste Anordnung (1) aus mindestens zwei Osteosyntheseplatten (2), zum Beispiel vom Typ I-form und/oder L-Form und/oder Kreuz-Form und/oder Gänsefuß-Form, die ihrerseits in Abhängigkeit von der durchzuführenden Operation und an die zukünftige Anatomie des Patienten angepasst ausgelegt sind, wobei die Osteosyntheseplatten (2) durch eine Stange (3) miteinander verbunden sind, **dadurch gekennzeichnet, dass** die Stange ferner zwei Vorsprünge (4) aufweist, die aus der Stange (3) in Richtung der Nasenöffnung (8) des Patienten an Positionen hervortreten, die den beiden Enden (5, 6) der Basis (7) der Nasenöffnung (8) des Patienten entsprechen.

2. Angepasste Anordnung (1) aus mindestens zwei Osteosyntheseplatten (2) nach Anspruch 1, **dadurch gekennzeichnet, dass** die zwei Vorsprünge (4) die Form von Zapfen oder Fingern oder Stiften oder Spornen aufweisen.

3. Angepasste Anordnung (1) aus mindestens zwei Osteosyntheseplatten (2) nach einem der Ansprüche 1 und 2, **dadurch gekennzeichnet, dass** auch die Stange (3) der Anordnung ihrerseits perfekt die Anatomie des Patienten berücksichtigt.

4. Angepasste Anordnung (1) aus mindestens zwei Osteosyntheseplatten (2) nach einem der Ansprüche 1 bis 3, **dadurch gekennzeichnet, dass** die Stange (3) der Anordnung nahe jeder ihrer Platten (2) Bereiche geringeren Widerstands (11) aufweist, die dazu dienen, das vom Chirurgen ausgeführte Durchschneiden aller Verbindungsteile zwischen den verschiedenen Osteosyntheseplatten der Anordnung zu erleichtern.

5. Angepasste Anordnung (1) aus mindestens zwei Osteosyntheseplatten (2) nach einem der Ansprüche 1 bis 4, **dadurch gekennzeichnet, dass** sie aus einem einzigen Teil gefertigt ist.

6. Angepasste Anordnung (1) aus mindestens zwei Osteosyntheseplatten (2) nach einem der Ansprüche 1 bis 5, **dadurch gekennzeichnet, dass** sie aus Titan gebildet ist.

## Claims

1. Customized assembly (1) including at least two osteosynthesis plates (2) which, for example, are of the I-shaped and/or L-shaped and/or cross-shaped and/or crow's foot-shaped types, and which are in turn customized according to the operation to be performed and according to the future anatomy of the patient, the osteosynthesis plates (2) being connected to each other by a rod (3), **characterized in that** said rod additionally has two protrusions (4) protruding from said rod (3) in the direction of the nasal orifice (8) of the patient, at positions that correspond to the two ends (5, 6) of the base (7) of the nasal orifice (8) of the patient.

2. Customized assembly (1) including at least two osteosynthesis plates (2) according to Claim 1, **characterized in that** the two protrusions (4) are in the form of stubs, fingers, lugs or spurs.

3. Customized assembly (1) including at least two osteosynthesis plates (2) according to either of Claims 1 and 2, **characterized in that** the rod (3) of said assembly also perfectly follows the anatomy of the patient.

4. Customized assembly (1) including at least two osteosynthesis plates (2) according to any one of Claims 1 to 3, **characterized in that**, close to each of its plates (2), the rod (3) of said assembly has zones of lesser resistance (11), which are designed to make it easier for the surgeon to cut all the connecting parts between the different osteosynthesis plates of said assembly.

5. Customized assembly (1) including at least two osteosynthesis plates (2) according to any one of Claims 1 to 4, **characterized in that** it is produced in one piece.

6. Customized assembly (1) including at least two osteosynthesis plates (2) according to any one of Claims 1 to 5, **characterized in that** it is made of titanium.
